# EUROPÄISCHE PATENTSCHRIFT

(11) **EP 4 168 063 B1**
(45) Veröffentlichungstag und Bekanntmachung des Hinweises auf die Patenterteilung: **18.03.2026**
(21) Anmeldenummer: 21733106.5
(22) Anmeldetag: 11.06.2021
(51) Int. Cl.: A61M 5/00, B65D 71/70

(54) **SCHALE FÜR DIE AUFNAHME EINER MEHRZAHL VON MEDIZINISCHEN HOHLKÖRPERN**
TRAY FOR RECEIVING A PLURALITY OF MEDICAL HOLLOW BODIES
PLATEAU POUR RECEVOIR UNE PLURALITÉ DE CORPS CREUX MÉDICAUX

(30) Priorität: 18.06.2020 DE 102020207588
(43) Veröffentlichungstag der Anmeldung: 26.04.2023
(73) Patentinhaber: Vetter Pharma-Fertigung GmbH & Co. KG, 88212 Ravensburg (DE)
(72) Erfinder: STEFAN, Ralph, 88370 Ebenweiler (DE); BEHRENDT, Markus, 24119 Kronshagen (DE); PUPKE, Holger, 72336 Balingen (DE); MOLLER, Karoline, 88214 Ravensburg (DE)
(74) Vertreter: Kordel, Mattias
(86) Internationale Anmeldenummer: PCT/EP2021/065868
(87) Internationale Veröffentlichungsnummer: WO 2021/254918

(56) Entgegenhaltungen:
- EP-A1- 0 790 063
- EP-A1- 3 437 678
- EP-B1- 0 790 063
- US-A1- 2004 238 391
- US-A1- 2013 062 245
- US-A1- 2016 015 457

## Beschreibung

Die Erfindung betrifft eine Schale für die Aufnahme einer Mehrzahl von medizinischen Hohlkörpern.

Aus dem US-amerikanischen Patent US 6,216,885 B1 ist eine Schale zur Aufnahme einer Mehrzahl von Spritzen bekannt, die aus einem thermisch geformten Kunststoffmaterial hergestellt und insbesondere tiefgezogen ist. Diese Schale weist eine sehr geringe Steifigkeit auf und ist deswegen schwierig zu handhaben. Um die Schale handhaben zu können, sind in regelmäßigen Abständen Greifstutzen vorgesehen, an denen beispielsweise Vakuumgreifer angreifen können, um die Schale zu halten. Die Schale ist auf ein bestimmtes Spritzenformat abgestimmt, wobei sie feste Klemmelemente aufweist, um die Spritzen des bestimmten Formats stabil und sicher zu halten. Die Schale ist somit nicht flexibel für verschiedene Spritzenformate verwendbar, und aufgrund der die Spritzen stabil fixierenden Klemmelemente ist es aufwendig, die Schale mit den Spritzen zu bestücken oder die Spritzen aus der Schale zu entnehmen.

Es sind auch Schalen bekannt, in die medizinische Hohlkörper lose eingelegt werden, was insbesondere nachteilig ist, wenn die medizinischen Hohlkörper Glas umfassen oder aus Glas bestehen. Es kommt dann zu Glas-Glas-Kontakt, was zu Beschädigungen insbesondere beim Transport führen kann. Insbesondere können dabei auch an den medizinischen Hohlkörpern angeordnete Etiketten beschädigt oder in Hinblick auf ihre Lesbarkeit beeinträchtigt werden. EP0790063, US2016015457 und US2013062245 beschreiben auch Schalen für die Aufnahme einer Mehrzahl von medizinischen Hohlkörpern nach dem Stand der Technik.

Der Erfindung liegt daher die Aufgabe zugrunde, eine Schale für die Aufnahme von medizinischen Hohlkörpern zu schaffen, bei der die genannten Nachteile zumindest teilweise behoben sind, wobei sie die Nachteile vorzugsweise nicht aufweist.

Die Aufgabe wird gelöst, indem die vorliegende technische Lehre bereitgestellt wird, insbesondere die Lehre der unabhängigen Ansprüche sowie der in den abhängigen Ansprüchen und der Beschreibung offenbarten Ausführungsformen.

Die Aufgabe wird insbesondere gelöst, indem eine Schale für die Aufnahme einer Mehrzahl von medizinischen Hohlkörpern geschaffen wird, die eine Bodenplatte aufweist. Die Bodenplatte weist eine Oberseite und eine Unterseite auf. Entlang äußerer Kanten der Schale ist an der Oberseite eine allseitig umlaufende Wandung angeordnet. Die Bodenplatte und die Wandung begrenzen ein Aufnahmevolumen zur Aufnahme der medizinischen Hohlkörper. Die Schale weist eine endliche Länge entlang einer Längsrichtung und eine endliche Breite entlang einer senkrecht auf der Längsrichtung stehenden Breitenrichtung auf. Die Schale weist wenigstens eine Haltestruktur zur Halterung medizinischer Hohlkörper auf. Die Haltestruktur wiederum weist eine erste Haltewand auf, die sich von einem ersten Breitenabschnitt der Wandung ausgehend bis zu einem gegenüberliegenden zweiten Breitenabschnitt der Wandung in Längsrichtung erstreckt. In der ersten Haltewand sind voneinander beabstandete erste Durchbrechungen ausgebildet. Die Haltestruktur weist außerdem eine von der ersten Haltewand durch einen ersten Breitenabstand beabstandete, sich parallel zu der ersten Haltewand von dem ersten Breitenabschnitt bis zu dem zweiten Breitenabschnitt erstreckende zweite Haltewand auf. In der zweiten Haltewand sind voneinander beabstandete zweite Durchbrechungen ausgebildet. Jede der zweiten Durchbrechungen fluchtet mit je einer ihr zugeordneten ersten Durchbrechung. Insbesondere sind die zweiten Durchbrechungen und die ersten Durchbrechungen einander paarweise zugeordnet, wobei die einander paarweise zugeordneten ersten und zweiten Durchbrechungen miteinander fluchten. Die Haltestruktur weist außerdem eine zu den Haltewänden durch einen zweiten Breitenabstand beabstandete, sich parallel zu den Haltewänden erstreckende Reihe von Abstandselementen auf, die voneinander unter Bildung einer Mehrzahl von Freiräumen beabstandet sind. Die Abstandselemente sind derart versetzt zu den Durchbrechungen angeordnet, dass jeder der Freiräume mit jeweils einer der ersten und der zweiten Durchbrechungen fluchtet. Jedem Paar aus eine ersten Durchbrechung und einer zweiten Durchbrechung ist somit ein Freiraum zugeordnet, wobei der entsprechende Freiraum mit der jeweiligen ersten Durchbrechung und mit der jeweiligen zweiten Durchbrechung des entsprechend zugeordneten Paars von ersten und zweiten Durchbrechungen fluchtet. Durch jeweils einen der Freiräume, die diesem Freiraum zugeordnete erste Durchbrechung und die dem Freiraum sowie der ersten Durchbrechung zugeordnete zweite Durchbrechung ist eine Halteposition für einen medizinischen Hohlkörper definiert, wobei sich die Halteposition in Breitenrichtung der Schale erstreckt. Der erste Breitenabstand ist kleiner als der zweite Breitenabstand.

Durch die geometrische Struktur mit der Bodenplatte und der allseitig umlaufenden Wandung, die gemeinsam das Aufnahmevolumen begrenzen, weist die Schale eine hohe Steifigkeit auf und ist somit zugleich einfach und stabil handhabbar. Es bedarf insbesondere keiner eigens hierfür vorzusehenden Greifstutzen, sodass hierfür andernfalls vorzusehendes Volumen eingespart und zur Aufnahme medizinischer Hohlkörper verwendet werden kann. Die Haltestruktur ist mit den durch die Durchbrechungen und Freiräume gebildeten Haltepositionen sehr flexibel und kann mit einer Vielzahl verschiedener Formate medizinischer Hohlkörper, insbesondere einer Vielzahl von Spritzenformaten - unabhängig davon, ob bereits eine Fingerauflage, Kolbenstange und/oder Injektionsnadel montiert ist/sind - verwendet werden. Zugleich verzichtet die Haltestruktur auf feste Klemmelemente, sodass die medizinischen Hohlkörper einfach und schnell, insbesondere manuell oder auch maschinell - insbesondere mittels Roboter -, in die Schale eingelegt und aus der Schale entnommen werden können. Die Schale ermöglicht also eine leichte Bestückung mit medizinischen Hohlkörpern sowie ebenfalls eine leichte Entnahme derselben. Zugleich stellen die Haltepositionen ein eindeutiges Einlegeschema für die Weiterverarbeitung der medizinischen Hohlkörper bereit. Weiterhin definieren die Haltepositionen eindeutige, voneinander beabstandete Bereiche für die einzelnen medizinischen Hohlkörper, sodass insbesondere Glas-Glas-Kontakt vermieden wird. Dadurch entfällt auch das Risiko einer Beschädigung der medizinischen Hohlkörper und/oder der daran gegebenenfalls angeordneten Etiketten. Insbesondere wird deren Lesbarkeit bei der Verwendung der hier vorgeschlagenen Schale nicht beeinträchtigt.

Insbesondere ist bei der hier vorgeschlagenen Schale eine genaue Anzahl eingelegter medizinischer Hohlkörper korrekt nachvollziehbar. Es können eine Vielzahl verschiedener Formate und Variationen medizinischer Hohlkörper mit einer einzigen Schale abgedeckt werden. Insbesondere können bei der Verwendung der hier vorgeschlagenen Schale verschiedene Spritzenformate mit und ohne Kolbenstange und/oder Fingerauflage vereinzelt werden. Durch den mittels der Haltepositionen bereitgestellten passgenauen Sitz ist ein Verrutschen der medizinischen Hohlkörper beim Transport ausgeschlossen.

Die Bodenplatte ist vorzugsweise flach und eben ausgebildet. Insbesondere ist die Bodenplatte frei von Ausstülpungen und/oder Ausformungen.

Insbesondere ist die Schale nicht tiefgezogen ausgebildet.

Vorzugsweise ist die Schalen mittels Spritzgießen hergestellt. Insbesondere durch die Robustheit des Spritzgussmaterials ist die Schale mehrfach verwendbar, insbesondere häufiger verwendbar als tiefgezogenes Material. Insbesondere kann die Schale als Umlaufmaterial verwendet werden.

Durch die Anordnung der Haltewände einerseits und der die Freiräume bildenden Abstandselemente andererseits ist ein falsches Einlegen der medizinischen Hohlkörper ausgeschlossen.

Die Wandung erstreckt sich vorzugsweise 90° zu der Bodenplatte. Insbesondere erstreckt sich die Wandung in einer Hochrichtung ausgehend von der Bodenplatte. Die Hochrichtung steht dabei senkrecht einerseits auf der Längsrichtung und andererseits auf der Breitenrichtung.

Die Längsrichtung ist bevorzugt eine Richtung längster Erstreckung der Schale. Die Breitenrichtung entspricht bevorzugt einer Richtung, in der sich die Schale über eine Breite erstreckt, die kürzer ist als die Länge, über die sich die Schale entlang der Längsrichtung erstreckt. Die Hochrichtung ist insbesondere eine Richtung, die sich bei bestimmungsgemäßer Anordnung der Schale in vertikaler Richtung erstreckt, also in geodätischer Hochrichtung. Die Längsrichtung und die Breitenrichtung erstrecken sich demgegenüber in einer Ebene, auf der die Hochrichtung, bei bestimmungsgemäßer Anordnung der Schale die Vertikale im Raum, senkrecht steht.

Die Wandung erstreckt sich in Hochrichtung bevorzugt bis mindestens zu einer Oberkante eines größten in das Aufnahmevolumen einlegbaren medizinischen Hohlkörpers, wenn dieser in das Aufnahmevolumen eingelegt ist. Vorzugsweise erstreckt sich die Wandung bis oberhalb der Oberkante des größten in das Aufnahmevolumen einzulegenden Hohlkörpers, wenn dieser in das Aufnahmevolumen eingelegt ist.

Die Haltewände sind vorzugsweise in der Hochrichtung genauso hoch wie die Wandung, erstrecken sich also insbesondere ausgehend von der Bodenplatte in Hochrichtung bis zu einer Oberkante der Wandung.

Die Durchbrechungen sind vorzugsweise nach oben hin offen, weisen also ein der Bodenplatte abgewandtes offenes Ende auf.

Die Schale ist insbesondere verwendbar mit folgenden Spitzenformaten: 1 ml, Durchmesser 8,15 mm, sowohl mit als auch ohne Kolbenstange/Fingerauflage, wobei die Glaskörperlänge mit Vetter RNS (Rigid Needle Shield) 81,2 mm und mit Vetter OVS 73,9 mm beträgt; 1,5 ml, Durchmesser 10,85 mm, sowohl mit als auch ohne Kolbenstange/Fingerauflage, mit einer Glaskörperlänge mit RNS 73,3 mm und mit Vetter OVS 63,5 mm; 2,25 mm, Durchmesser 10,85 mm, sowohl mit als auch ohne Kolbenstange/Fingerauflage, wobei die Glaskörperlänge mit Vetter RNS 82,8 mm und mit Vetter OVS 75 mm beträgt. Als Verschlussteile können Vetter OVS, Vetter TipCap, Vetter NS (Needle Shield) und Vetter RNS verwendet werden.

Die in Längsrichtung gemessene Länge der Schale beträgt bevorzugt von mindestens 300 mm bis höchstens 400 mm, vorzugsweise von mindestens 325 mm bis höchstens 375 mm, vorzugsweise 350 mm.

Die in Breitenrichtung gemessene Breite der Schale beträgt bevorzugt von mindestens 200 mm bis höchstens 300 mm, vorzugsweise von mindestens 225 mm bis höchstens 295 mm, vorzugsweise von mindestens 250 mm bis höchstens 280 mm, vorzugsweise von mindestens 265 mm bis höchstens 275 mm, vorzugsweise 270 mm.

Die Schale ist bevorzugt als Wanneneinlage zum Einsetzen oder Einlegen in eine größere Transportwanne ausgebildet, wobei in die Transportwanne eine Mehrzahl solcher Schalen eingelegt oder eingesetzt werden kann. Die Maße der Schale, insbesondere deren Länge und deren Breite, sind vorzugsweise so bemessen, dass zwei solche Schalen nebeneinander in die Transportwanne eingelegt werden können. Die Höhe der Schale ist insbesondere so bemessen, dass eine Mehrzahl von Schalen, insbesondere mehr als zwei Schalen, vorzugsweise vier oder mehr Schalen, übereinander in eine solche Transportwanne eingelegt werden können.

Eine in Hochrichtung gemessene Höhe der Schale beträgt von einer Unterkante der Bodenplatte bis zu einer Oberkante der Wandung von mindestens 15 mm bis höchstens 30 mm, vorzugsweise von mindestens 17,5 mm bis höchstens 25 mm, vorzugsweise von mindestens 20 mm bis höchstens 23 mm, vorzugsweise 22 mm. Weist die Schale zusätzlich Fußelemente auf, beträgt ihre Höhe einschließlich der Fußelemente bevorzugt von mindestens 23 mm bis höchstens 30 mm, vorzugsweise von mindestens 24 mm bis höchstens 28 mm, vorzugsweise 26 mm.

Der erste Breitenabstand beträgt bevorzugt von mindestens 6 mm bis höchstens 12 mm, vorzugsweise 9 mm. Der zweite Breitenabstand beträgt bevorzugt von mindestens 26 mm bis höchstens 30 mm, vorzugsweise 28 mm. Der dritte Breitenabstand beträgt bevorzugt von mindestens 36 mm bis höchstens 42 mm, vorzugsweise 39 mm. Ein einzelner Freiraum der Freiräume weist bevorzugt eine in Längsrichtung der Schale gemessene Erstreckung, d.h. lichte Weite, von mindestens 6 mm bis höchstens 12 mm, vorzugsweise 9 mm, auf. Bevorzugt sind alle Freiräume identisch ausgebildet.

Gemäß einer Weiterbildung der Erfindung ist vorgesehen, dass die Schale als die wenigstens eine Haltestruktur zwei Haltestrukturen aufweist, die in Breitenrichtung nebeneinander angeordnet und durch eine sich in Längsrichtung von dem ersten Breitenabschnitt zu dem zweiten Breitenabschnitt erstreckende Trennwand voneinander getrennt sind. Die Schale weist also insgesamt zwei Haltestrukturen auf, wobei insbesondere auch jeweils zwei Haltepositionen in Breitenrichtung nebeneinander angeordnet sind. Die Schale kann somit zwei Reihen medizinischer Hohlkörper in Breitenrichtung nebeneinander aufnehmen, was bedeutet, dass die Hohlkörper der beiden Reihen jeweils paarweise in Längsrichtung der Hohlkörper gesehen hintereinander in der Schale angeordnet sind. Die Schale kann auf diese Weise sehr funktionell eine Vielzahl medizinischer Hohlkörper geordnet aufnehmen.

Die Trennwand erstreckt sich vorzugsweise in Hochrichtung bis zu der Oberkante der Wandung, ist also genauso hoch ausgebildet wie die Wandung.

Gemäß einer Weiterbildung der Erfindung ist vorgesehen, dass die erste Haltewand der wenigstens einen Haltestruktur von einer Stirnwand, die ausgewählt ist aus einem Längsabschnitt der Wandung und der Trennwand, durch einen dritten Breitenabstand beabstandet ist. Dies gilt vorzugsweise für beide Haltestrukturen der Schale, wenn diese zwei Haltestrukturen aufweist. Dabei ist die Stirnwand bei der einen Haltestruktur der beiden Haltestrukturen der Längsabschnitt der Wandung; bei der anderen Haltestruktur der beiden Haltestrukturen ist die Stirnwand die Trennwand, welche die beiden Haltestrukturen voneinander trennt. In vorteilhafter Weise kann in einem Bereich, der zwischen der ersten Haltewand und der Stirnwand angeordnet ist, eine Kolbenstange eines medizinischen Hohlkörpers aufgenommen sein, wenn der medizinische Hohlkörper eine solche Kolbenstange aufweist.

Gemäß einer Weiterbildung der Erfindung ist vorgesehen, dass die Breitenabschnitte der Wandung jeweils zwischen der zweiten Haltewand und der Reihe von Abstandselementen eine Aussparung aufweisen. Diese Aussparung ermöglicht in vorteilhafter Weise ein einfaches Eingreifen mit einem Greifer oder auch manuell mit der Hand, beispielsweise mit einem Finger, um insbesondere auch randständig angeordnete medizinische Hohlkörper sicher und leicht in die Schale einlegen und wieder Entnehmen zu können.

Die Aussparungen sind vorzugsweise nach oben offen, weisen also ein der Bodenplatte abgewandtes offenes Ende auf.

Gemäß einer Weiterbildung der Erfindung ist vorgesehen, dass die Bodenplatte im Bereich der Reihe von Abstandselementen eine Erhebung aufweist. Insbesondere kann die Bodenplatte im Bereich der Abstandselemente eine von der Ebene abweichende Form und insbesondere eine Ausbuchtung nach oben aufweisen. Die Abstandselemente sind auf der Erhebung angeordnet. Die Erhebung stellt im Bereich der Freiräume einen von einer übrigen Bodenfläche der Schale abgesetzten Auflagebereich für die medizinischen Hohlkörper bereit, sodass diese insbesondere im Bereich zwischen der zweiten Haltewand und der Reihe von Abstandselementen nicht auf der Bodenfläche aufliegen. Die Hohlkörper können dann dort besonders einfach erfasst, insbesondere gegriffen werden. Die Erhebung führt außerdem strukturell zu einer Versteifung der Schale, sodass diese eine besonders hohe Stabilität aufweist. An der Unterseite der Bodenplatte stellt die oberseitige Erhebung eine Vertiefung dar, die wiederum ein besonders einfaches Greifen und Handhaben der Schale selbst, sei es manuell oder maschinell, ermöglicht. Insbesondere bildet die oberseitig ausgebildete Erhebung unterseitig eine Eingreifnut, mit der insbesondere ein leichtes Entnehmen der Schale aus einer Transportwanne möglich ist.

Gemäß einer Weiterbildung der Erfindung ist vorgesehen, dass die Abstandselemente in Draufsicht gesehen länglich ausgebildet sind. Dies stellt eine besonders einfache und einfach herzustellende Ausgestaltung der Abstandselemente dar.

Alternativ ist bevorzugt vorgesehen, dass die Abstandselemente in Draufsicht gesehen kreuzförmig ausgebildet sind. Die Abstandselementen erfüllen damit eine Zusatzfunktion zusätzlich zum Beabstanden der medizinischen Hohlkörper, indem sie aufgrund ihrer kreuzförmigen Struktur zur Versteifung und damit zur Stabilität der Schale beitragen. Insbesondere aufgrund dieser Strukturverstärkung ist eine hohe Stapelung einer Vielzahl von Schalen aufeinander möglich.

Gemäß einer Weiterbildung der Erfindung ist vorgesehen, dass die Bodenplatte an ihrer Unterseite Fußelemente aufweist. In bevorzugter Ausgestaltung kann die Schale an der Unterseite der Bodenplatte vier Fußelemente, insbesondere an jeder Ecke ein Fußelement, aufweisen. Die Fußelemente ragen von der Unterseite - geodätisch nach unten - ab. Sie bilden somit Aufstandselemente, auf denen die Schale aufstehen kann, wobei die Unterseite der Bodenplatte dann von einer Aufstandsebene beabstandet ist, auf der die Schale aufsteht.

Bevorzugt sind die Fußelemente von den äußeren Kanten der Bodenplatte um mindestens eine Wandungsstärke der Wandung nach innen versetzt angeordnet. Besonders bevorzugt sind sie genau um die Wandungsstärke der Wandung nach innen versetzt angeordnet. Insbesondere dies erlaubt eine stabile Stapelung einer Mehrzahl von Schalen übereinander, wobei jeweils die Fußelemente einer obenliegenden Schale in das Aufnahmevolumen der darunterliegenden Schale eingreifen und dabei bevorzugt stabil innenseitig an der Wandung anliegen. Durch diesen insbesondere passgenauen Sitz der Fußelemente ist bevorzugt ein Verrutschen der gestapelten Schalen relativ zueinander wirksam verhindert.

Gemäß einer Weiterbildung der Erfindung ist vorgesehen, dass die Schale einteilig und materialeinheitlich ausgebildet ist die Schale kann auf diese Weise besonders einfach und kostengünstig hergestellt sein, insbesondere durch Spritzgießen.

Die Schale ist bevorzugt einteilig und materialeinheitlich ausgebildet, vorzugsweise aus einem Kunststoff. Die Schale kann so besonders einfach und kostengünstig hergestellt sein, insbesondere durch Spritzgießen. Der Kunststoff ist bevorzugt ausgewählt aus einer Gruppe, bestehend aus: Einem autoklavierbaren Kunststoff, einem maschinenspülbaren Kunststoff, Acrylnitril-Butadien-Styrol-Copolymer (ABS), Polycarbonat, und Polypropylen. Insbesondere diese Kunststoffe erlauben eine einfache Reinigung der Schale, insbesondere mittels Spülmaschine, wobei die Schale bevorzugt auch autoklavierbar ist. Insbesondere dies ermöglicht eine Wiederverwendung der Schale, insbesondere eine Verwendung der Schale im Umlaufverfahren.

Zu Erfindung gehört auch die erfindungsgemäße Schale oder eine Schale nach einem der zuvor beschriebenen Ausführungsbeispiele in Kombination mit einem medizinischen Hohlkörper, insbesondere einem der oben beschriebenen medizinischen Hohlkörper, mit denen die Schale bevorzugt verwendet werden kann. Der medizinische Hohlkörper weist einen Zylinderabschnitt und einen Flanschbereich auf. Wenn der medizinische Hohlkörper in der Schale angeordnet ist, ist der Flanschbereich zwischen der ersten Haltewand der zweiten Haltewand angeordnet. Dabei hintergreift der Flanschbereich bevorzugt eine dem medizinischen Hohlkörper zugeordnete zweite Durchbrechung der zweiten Durchbrechungen. Der Zylinderabschnitt erstreckt sich in Breitenrichtung der Schale durch die zweite Durchbrechung hindurch und in den Freiraum hinein, der derjenigen zweiten Durchbrechung zugeordnet ist, die dem medizinischen Hohlkörper zugeordnet ist. Auf diese Weise ist der medizinische Hohlkörper sicher und stabil in der Schale gehalten. Insbesondere kann er in der Schale nicht verrutschen und nicht in Kontakt mit anderen in der Schale aufgenommenen medizinischen Hohlkörpern kommen. Auf diese Weise wird insbesondere ein Glas-Glas-Kontakt zwischen den in der Schale angeordneten medizinischen Hohlkörper vermieden.

Der Flansch des medizinischen Hohlkörpers kann insbesondere eine Fingerauflage oder ein Backstop sein. Der medizinische Hohlkörper ist - insbesondere je nach seinem Außendurchmesser - zumindest in dem Freiraum, gegebenenfalls auch zumindest in der zweiten Durchbrechung, mit Spiel angeordnet. Die Maße der Schale sind dabei bevorzugt derart auf einen größten in der Schale anzuordnenden medizinischen Hohlkörper abgestimmt, dass dieser gerade ohne feste Klemmung, vorzugsweise mit geringem Spiel, an der Halteposition angeordnet werden kann. Ist der medizinische Hohlkörper als Spritze ausgebildet, ist ein Injektionsende der Spritze bevorzugt zwischen der Reihe von Abstandselementen und einer hinteren Stirnwand angeordnet, wobei die hintere Stirnwand wiederum ausgewählt ist aus einem Längsabschnitt der Wandung und der Trennwand, je nachdem welcher Haltestruktur die Reihe von Abstandselementen zugeordnet ist. Die Trennwand ist somit insbesondere für eine erste Haltestruktur der beiden Haltestrukturen die hintere Stirnwand, und für eine zweite Haltestruktur der beiden Haltestrukturen die vordere Stirnwand.

Gemäß einer Weiterbildung der Erfindung ist vorgesehen, dass sich eine Kolbenstange des medizinischen Hohlkörpers durch die erste Durchbrechung hindurch in Richtung der Stirnwand erstreckt. Somit steht der dritte Breitenabstand insbesondere zur Anordnung der Kolbenstange zur Verfügung.

Die Erfindung wird im Folgenden anhand der Zeichnung näher erläutert. Dabei zeigen:
- Figur 1: ein erstes Ausführungsbeispiel einer Schale;
- Figur 2: eine Querschnittsdarstellung des ersten Ausführungsbeispiels der Schale entlang der in Figur 1 dargestellten Linie A-A, und
- Figur 3: eine Darstellung einer Mehrzahl übereinander gestapelter Schalen gemäß einem zweiten Ausführungsbeispiel in Kombination mit medizinischen Hohlkörpern.

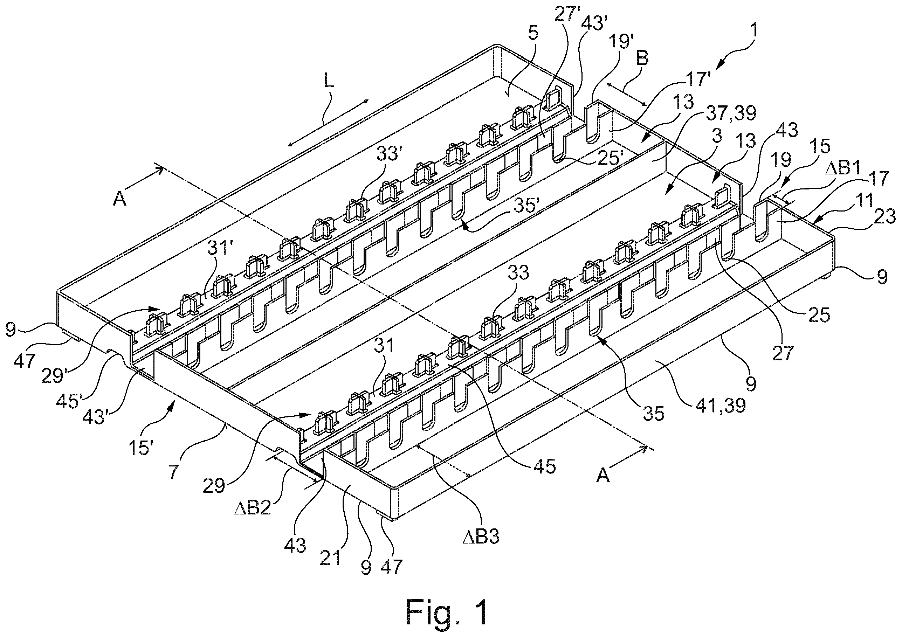

**Fig. 1** zeigt eine Darstellung eines ersten Ausführungsbeispiels einer Schale 1, die eingerichtet ist, um eine Mehrzahl von medizinischen Hohlkörpern aufzunehmen. Die Schale 1 weist eine Bodenplatte 3 auf, die eine Oberseite 5 und eine Unterseite 7 aufweist. Entlang äußerer Kanten 9 der Schale 1 ist an der Oberseite 5 eine allseitig umlaufende Wandung 11 angeordnet. Die Bodenplatte 3 und die Wandung 11 begrenzen ein Aufnahmevolumen 13 zur Aufnahme der medizinischen Hohlkörper. Die Schale 1 weist eine endliche Länge entlang einer Längsrichtung L und eine endliche Breite entlang einer senkrecht auf der Längsrichtung L stehenden Breitenrichtung B auf. Die Schale 1 weist außerdem eine Haltestruktur 15 zur Halterung medizinischer Hohlkörper auf.

Insbesondere weist die Schale 1 hier eine erste Haltestruktur 15 und eine zweite Haltestruktur 15° auf. Die beiden Haltestrukturen 15,15' sind identisch ausgebildet, sodass insofern im Folgenden lediglich auf die Ausgestaltung der ersten Haltestruktur 15 näher eingegangen wird. Die entsprechenden Elemente der zweiten Haltestruktur 15' sind jeweils mit gestrichenen Bezugszeichen dargestellt.

Die Haltestruktur 15 weist eine erste Haltewand 17 auf, die sich von einem ersten Breitenabschnitt 21 der Wandung 11 ausgehend bis zu einem gegenüberliegenden zweiten Breitenabschnitt 23 der Wandung 11 in Längsrichtung L erstreckt. Dabei sind in der ersten Haltewand 17 erste Durchbrechungen 25 ausgebildet, die voneinander beabstandet sind. Die Haltestruktur 17 weist außerdem eine zweite Haltewand 19 auf, die von der ersten Haltewand 17 durch einen ersten Breitenabstand ΔB1 beabstandet ist. Die zweite Haltewand 19 erstreckt sich dabei parallel zu der ersten Haltewand 17 von dem ersten Breitenabschnitt 21 bis zu dem zweiten Breitenabschnitt 23. In der zweiten Haltewand 19 sind zweite Durchbrechungen 27 ausgebildet, die voneinander beabstandet sind. Dabei fluchtet jede der zweiten Durchbrechungen 27 mit je einer ihr zugeordneten ersten Durchbrechung 25. Die Haltestruktur 15 weist außerdem eine zu den Haltewänden 17, 19 durch einen zweiten Breitenabstand ΔB2 beabstandete Reihe 29 von Abstandselementen 33 auf, wobei sich die Reihe 29 parallel zu den Haltewänden 17, 19 erstreckt, und wobei die Abstandselemente 33 unter Bildung einer Mehrzahl von Freiräumen 31 voneinander beabstandet sind. Die Abstandselemente 33 sind derart versetzt zu den Durchbrechungen 25, 27 angeordnet, dass jeder der Freiräume 31 mit jeweils einer der ersten Durchbrechungen 25 und der zweiten Durchbrechungen 27 fluchtet. Auf diese Weise ist durch jeweils einen der Freiräume 31, die ihm zugeordnete erste Durchbrechung 25 und die ihm zugeordnete zweite Durchbrechung 27 eine Halteposition 35 für einen medizinischen Hohlkörper definiert, wobei sich die Halteposition 35 in Breitenrichtung B erstreckt.

Der erste Breitenabstand ΔB1 ist kleiner als der zweite Breitenabstand ΔB2.

Die erste Haltestruktur 15 und die zweite Haltestruktur 15' sind in Breitenrichtung B nebeneinander angeordnet und durch eine sich in Längsrichtung L von dem ersten Breitenabschnitt 21 zu dem zweiten Breitenabschnitt 23 erstreckende Trennwand 37 voneinander getrennt.

Die erste Haltewand 17 ist von einer Stirnwand 39 durch einen dritten Breitenabstand ΔB3 beabstandet. Die Stirnwand 39 ist in diesem Fall ein Längsabschnitt 41 der Wandung 11. Die erste Haltewand 17' der zweiten Haltestruktur 15' ist ebenfalls von einer weiteren Stirnwand 39' durch den dritten Breitenabstand ΔB3 beabstandet. In diesem Fall ist die weitere Stirnwand 39' die Trennwand 37.

Die Breitenabschnitte 21, 23 der Wandung 11 weisen jeweils zwischen der zweiten Haltewand 19 und der Reihe 29 von Abstandselementen 33 eine Aussparung 43 auf.

Die Bodenplatte 3 weist im Bereich der Reihe 29 von Abstandselementen 33 eine Erhebung 45 auf. Die Abstandselemente 33 sind dabei auf der Erhebung 45 angeordnet.

Bei dem in Figur 1 dargestellten, ersten Ausführungsbeispiel sind die Abstandselemente 33 in Draufsicht kreuzförmig ausgebildet.

Die Bodenplatte 3 weist an ihrer Unterseite 7 Fußelemente 47 auf, die von der Unterseite 7 abragen. Dabei sind die Fußelemente 47 vorzugsweise von den äußeren Kanten 9 der Bodenplatte 3 um mindestens eine Wandungsstärke der Wandung 11, vorzugsweise um die Wandungsstärke, nach innen versetzt angeordnet.

Die Schale 1 ist insbesondere einteilig und materialeinheitlich ausgebildet, vorzugsweise aus einem Kunststoff. Der Kunststoff ist bevorzugt ausgewählt aus einer Gruppe, bestehend aus: Einem autoklavierbaren Kunststoff, einem maschinenspülbaren Kunststoff, Acrylnitril-Butadien-Styrol-Copolymer, Polycarbonat, und Polypropylen.

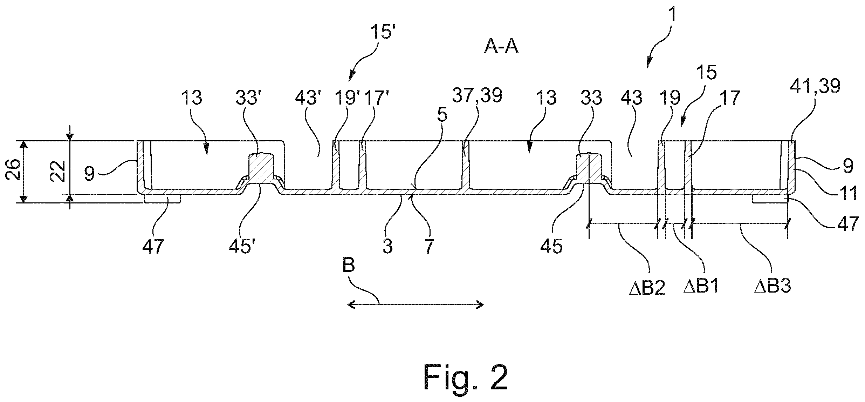

**Fig. 2** zeigt eine Querschnittsansicht der Schale 1 gemäß dem ersten Ausführungsbeispiel entlang der in Figur 1 dargestellten Linie A-A. Dabei sind in Figur 2 insbesondere der erste Breitenabstand ΔB1, der zweite Breitenabstand ΔB2 unter dritte Breitenabstand ΔB3 eingezeichnet. Außerdem ist in Figur 2 eingezeichnet, dass die in Hochrichtung gemessene Höhe der Schale 1 einschließlich der Fußelemente 47 26 mm beträgt, wobei die Höhe abzüglich der Fußelemente 47 22 mm beträgt.

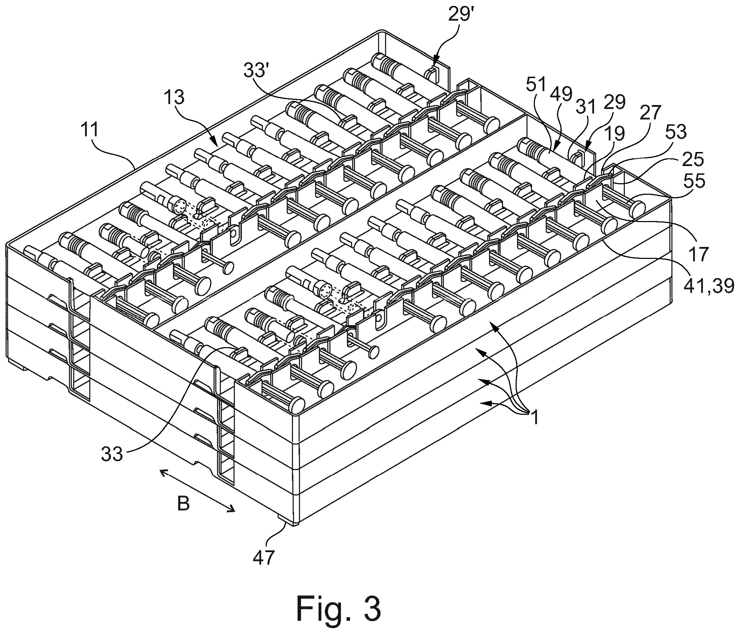

**Fig. 3** zeigt eine Darstellung einer Mehrzahl übereinander gestapelter Schalen 1 gemäß einem zweiten Ausführungsbeispiel in Kombination mit einer Mehrzahl von medizinischen Hohlkörpern 49. Gleiche und funktionsgleiche Elemente sind mit gleichen Bezugszeichen versehen, sodass insofern auf die vorangegangene Beschreibung verwiesen wird. Der übersichtlichen Darstellung wegen ist allerdings nur einer der Hohlkörper 49 mit dem entsprechenden Bezugszeichen sowie auch mit weiteren, den Hohlkörper 49 betreffenden Bezugszeichen bezeichnet.

Insbesondere die Fußelemente 47 ermöglichen ein stabiles Stapeln der Mehrzahl von Schalen 1 übereinander, ohne dass die Gefahr besteht, dass die einzelnen Schalen 1 relativ zueinander verrutschen können. Dazu greifen insbesondere die Fußelemente 47 der jeweils obenliegenden Schalen 1 in die Aufnahmevolumina 13 der jeweils darunterliegenden Schalen 1 ein und liegen bevorzugt innenseitig an den jeweiligen Wandungen 11 an.

Bei diesem zweiten Ausführungsbeispiel der Schale 1 sind die Abstandselemente 33, 33' in Draufsicht gesehen länglich ausgebildet.

Die medizinischen Hohlkörper 49 sind hier insbesondere als Spritzen ausgebildet. Im Folgenden wird nur einer der medizinischen Hohlkörper 49 näher beschrieben. Die medizinischen Hohlkörper 49 sind vorzugsweise insoweit identisch oder zumindest ähnlich ausgestaltet. Der hier näher betrachtete medizinische Hohlkörper 49 weist einen Zylinderabschnitt 51 und einen Flanschbereich 53 auf, wobei der Flanschbereich 53 zwischen der ersten Haltewand 17 und der zweiten Haltewand 19 angeordnet ist. Dabei hintergreift er eine dem medizinischen Hohlkörper 49 zugeordnete zweite Durchbrechung 27. Der Zylinderabschnitt 51 erstreckt sich in Breitenrichtung B der Schale 1 durch die zweite Durchbrechung 27 hindurch und in den Freiraum 31 hinein, der der zweiten Durchbrechungen 27 zugeordnet ist, die ihrerseits wiederum dem medizinischen Hohlkörper 49 zugeordnet ist.

Eine Kolbenstange 55 des medizinischen Hohlkörpers 49 erstreckt sich durch die erste Durchbrechung 25 hindurch in Richtung der Stirnwand 39.

Der Flanschbereich 53 ist vorzugsweise als Fingerauflage oder als Backstop ausgebildet.

Abweichend von der hier gewählten Darstellung können aber auch medizinische Hohlkörper 49 in der Schale 1 aufgenommen werden, die noch nicht montiert sind, also insbesondere noch keinen Flanschbereich 53 und/oder keine Kolbenstange 55 aufweisen.

## Patentansprüche

1. Schale (1) für die Aufnahme einer Mehrzahl von medizinischen Hohlkörpern (49), mit
- einer Bodenplatte (3), die eine Oberseite (5) und eine Unterseite (7) aufweist, wobei
- entlang äußerer Kanten (9) der Schale (1) an der Oberseite (5) eine allseitig umlaufende Wandung (11) angeordnet ist, wobei
- die Bodenplatte (3) und die Wandung (11) ein Aufnahmevolumen (13) zur Aufnahme der medizinischen Hohlkörper (49) begrenzen, wobei
- die Schale (1) eine endliche Länge entlang einer Längsrichtung (L) und eine endliche Breite entlang einer senkrecht auf der Längsrichtung (L) stehenden Breitenrichtung (B) aufweist, wobei
- die Schale (1) wenigstens eine Haltestruktur (15) zur Halterung medizinischer Hohlkörper (49) aufweist, wobei die wenigstens eine Haltestruktur (15) aufweist:
∘ eine erste Haltewand (17), die sich von einem ersten Breitenabschnitt (21) der Wandung (11) ausgehend bis zu einem gegenüberliegenden zweiten Breitenabschnitt (23) der Wandung (11) in Längsrichtung (L) erstreckt, wobei in der ersten Haltewand (17) voneinander beabstandete erste Durchbrechungen (25) ausgebildet sind,
∘ eine von der ersten Haltewand (17) durch einen ersten Breitenabstand (ΔB1) beabstandete, sich parallel zu der ersten Haltewand (17) von dem ersten Breitenabschnitt (21) bis zu dem zweiten Breitenabschnitt (23) erstreckende zweite Haltewand (19), wobei in der zweiten Haltewand (19) voneinander beabstandete zweite Durchbrechungen (27) ausgebildet sind, wobei jede der zweiten Durchbrechungen (27) mit je einer zugeordneten ersten Durchbrechung (25) fluchtet, und
∘ eine zu den Haltewänden (17,19) durch einen zweiten Breitenabstand (ΔB2) beabstandete, sich parallel zu den Haltewänden (17,19) erstreckende Reihe (29) voneinander unter Bildung einer Mehrzahl von Freiräumen (31) beabstandeter Abstandselemente (33), wobei die Abstandselemente (33) derart versetzt zu den Durchbrechungen (25,27) angeordnet sind, dass jeder der Freiräume (31) mit jeweils einer der ersten und der zweiten Durchbrechungen (25,27) fluchtet, so dass
∘ jeweils durch einen der Freiräume (31), eine zugeordnete erste Durchbrechung (25) und eine zugeordnete zweite Durchbrechung (27) eine Halteposition (35) für einen medizinischen Hohlkörper (49) definiert ist, die sich in Breitenrichtung (B) erstreckt, und wobei
∘ der erste Breitenabstand (ΔB1) kleiner ist als der zweite Breitenabstand (ΔB2).

2. Schale (1) nach Anspruch 1, wobei die Schale (1) als die wenigstens eine Haltestruktur (15) zwei Haltestrukturen (15,15') aufweist, die in Breitenrichtung (B) nebeneinander angeordnet und durch eine sich in Längsrichtung (L) von dem ersten Breitenabschnitt (21) zu dem zweiten Breitenabschnitt (23) erstreckende Trennwand (37) voneinander getrennt sind.

3. Schale (1) nach einem der vorhergehenden Ansprüche, wobei die erste Haltewand (17) der wenigstens einen Haltestruktur (15) von einer Stirnwand (39), ausgewählt aus einem Längsabschnitt (41) der Wandung (11) und der Trennwand (37), durch einen dritten Breitenabstand (ΔB3) beabstandet ist.

4. Schale (1) nach einem der vorhergehenden Ansprüche, wobei die Breitenabschnitte (21,23) der Wandung (11) jeweils zwischen der zweiten Haltewand (19) und der Reihe (29) von Abstandselementen (33) eine Aussparung (43) aufweisen.

5. Schale (1) nach einem der vorhergehenden Ansprüche, wobei die Bodenplatte (3) im Bereich der Reihe (29) von Abstandselementen (33) eine Erhebung (45) aufweist, wobei die Abstandselemente (33) auf der Erhebung (45) angeordnet sind.

6. Schale (1) nach einem der vorhergehenden Ansprüche, wobei die Abstandselemente (33) in Draufsicht länglich oder kreuzförmig ausgebildet sind.

7. Schale (1) nach einem der vorhergehenden Ansprüche, wobei die Bodenplatte (3) an der Unterseite (7) Fußelemente (47) aufweist, die von der Unterseite (7) abragen und vorzugsweise von den äußeren Kanten (9) der Bodenplatte (3) um mindestens eine Wandungsstärke der Wandung (11), vorzugsweise um die Wandungsstärke, nach innen versetzt angeordnet sind.

8. Schale (1) nach einem der vorhergehenden Ansprüche, wobei die Schale (1) einteilig und materialeinheitlich ausgebildet ist, vorzugsweise aus einem Kunststoff, wobei der Kunststoff vorzugsweise ausgewählt ist aus einer Gruppe, bestehend aus: Einem autoklavierbaren Kunststoff, einem maschinenspülbaren Kunststoff, Acrylnitril-Butadien-Styrol-Copolymer, Polycarbonat, und Polypropylen.

9. Schale (1) nach einem der vorhergehenden Ansprüche in Kombination mit einem medizinischen Hohlkörper (49), wobei der medizinische Hohlkörper (49) einen Zylinderabschnitt (51) und einen Flanschbereich (53) aufweist, wobei der Flanschbereich (53) zwischen der ersten Haltewand (17) und der zweiten Haltewand (19) angeordnet ist und vorzugsweise eine dem medizinischen Hohlkörper (49) zugeordnete zweite Durchbrechung (27) der zweiten Durchbrechungen (27) hintergreift, wobei sich der Zylinderabschnitt (51) in Breitenrichtung (B) der Schale (1) durch die zweite Durchbrechung (27) hindurch und in den der dem medizinischen Hohlkörper (49) zugeordneten zweiten Durchbrechung (27) zugeordneten Freiraum (31) hinein erstreckt.

10. Schale (1) nach Anspruch 9 in Kombination mit dem medizinischen Hohlkörper (49), wobei sich eine Kolbenstange (55) des medizinischen Hohlkörpers (49) durch die erste Durchbrechung (25) hindurch in Richtung der Stirnwand (39) erstreckt.

## Claims

1. Tray (1) for holding a plurality of medical hollow bodies (49), comprising
- a bottom plate (3) having a top side (5) and a bottom side (7), wherein
- an all-around wall (11) is disposed along outer edges (9) of the tray (1) on the top side (5), wherein
- the bottom plate (3) and the wall (11) delimit a receiving volume (13) for receiving the medical hollow bodies (49), wherein
- the tray (1) has a finite length along a longitudinal direction (L) and a finite width along a width direction (B) perpendicular to the longitudinal direction (L), wherein
- the tray (1) comprises at least one holding structure (15) for holding medical hollow bodies (49), wherein the at least one holding structure (15) comprises:
∘ a first holding wall (17) extending from a first width portion (21) of the wall (11) to an opposite second width portion (23) of the wall (11) in the longitudinal direction (L), spaced first breakthroughs (25) being formed in the first holding wall (17),
∘ a second holding wall (19) spaced from said first holding wall (17) by a first width distance (ΔB1) and extending parallel to said first holding wall (17) from said first width portion (21) to said second width portion (23), said second holding wall (19) having formed therein spaced second breakthroughs (27), each of said second breakthroughs (27) being aligned with an associated one of said first breakthroughs (25), and
∘ a row (29) of distance elements (33) spaced from the first holding walls (17,19) by a second width distance (ΔB2) and extending parallel to the first holding walls (17,19) to form a plurality of spaces (31), the distance elements (33) being arranged offset from the first breakthroughs (25,27) such that each of the spaces (31) is aligned with a respective one of the first and second breakthroughs (25,27) so that
∘ a holding position (35) for a medical hollow body (49) is defined by a respective one of said spaces (31), an associated first breakthrough (25) and an associated second breakthrough (27), said holding position (35) extending in the width direction (B), and wherein
∘ the first width distance (ΔB1) is smaller than the second width distance (ΔB2).

2. Tray (1) according to claim 1, wherein the tray (1) comprises as the at least one holding structure (15) two holding structures (15,15') arranged side by side in the width direction (B) and separated from each other by a separating wall (37) extending in longitudinal direction (L) from the first width portion (21) to the second width portion (23).

3. Tray (1) according to one of the preceding claims, wherein the first holding wall (17) of the at least one holding structure (15) is spaced from an end wall (39) selected from a longitudinal portion (41) of the wall (11) and the separating wall (37) by a third width distance (ΔB3).

4. Tray (1) according to one of the preceding claims, wherein the width portions (21,23) of the wall (11) each comprise a recess (43) between the second holding wall (19) and the row (29) of distance elements (33).

5. Tray (1) according to one of the preceding claims, wherein the bottom plate (3) has an elevation (45) in the region of the row (29) of distance elements (33), wherein the distance elements (33) are arranged on the elevation (45).

6. Tray (1) according to one of the preceding claims, wherein the distance elements (33) are elongated or cross-shaped in top view.

7. Tray (1) according to one of the preceding claims, wherein the bottom plate (3) has foot elements (47) on the bottom side (7), which protrude from the bottom side (7) and are preferably arranged offset inwards from the outer edges (9) of the bottom plate (3) by at least one wall thickness of the wall (11), preferably by the wall thickness.

8. Tray (1) according to one of the preceding claims, wherein the tray (1) is formed in one piece and of uniform material, preferably of a plastic, wherein the plastic is preferably selected from a group consisting of: An autoclavable plastic, a machine rinsable plastic, acrylonitrile-butadiene-styrene-copolymer, polycarbonate, and polypropylene.

9. Tray (1) according to one of the preceding claims in combination with a medical hollow body (49), wherein the medical hollow body (49) comprises a cylinder portion (51) and a flange area (53), wherein the flange area (53) is arranged between the first holding wall (17) and the second holding wall (19) and preferably engages behind a second breakthrough (27) of the second breakthroughs (27) associated with the medical hollow body (49), wherein the cylinder portion (51) extends in the width direction (B) of the tray (1) through the second breakthrough (27) and into the spaces (31) associated with the second breakthrough (27) associated with the medical hollow bodies (49).

10. Tray (1) according to claim 9 in combination with the medical hollow bodies (49), wherein a piston rod (55) of the medical hollow bodies (49) extends through the first breakthrough (25) towards the end wall (39).

## Revendications

1. Plateau (1) pour recevoir une pluralité de corps creux médicaux (49), avec
- une plaque de fond (3) présentant une face supérieure (5) et une face inférieure (7), dans lequel
- une paroi circonférentielle (11) est disposée le long de bords extérieurs (9) du plateau (1) au niveau de la face supérieure (5), dans lequel
- la plaque de fond (3) et la paroi (11) délimitent un volume de réception (13) prévu pour la réception des corps creux médicaux (49), dans lequel
- le plateau (1) présente une longueur finie dans une direction longitudinale (L) et une largeur finie dans une direction de largeur (B) perpendiculaire à la direction longitudinale (L), dans lequel
- le plateau (1) présente au moins une structure de maintien (15) pour maintenir des corps creux médicaux (49), dans lequel l'au moins une structure de maintien présente:
∘ une première cloison de maintien (17) qui s'étend dans la direction longitudinale (L) depuis une première section de largeur (21) de la paroi (11) jusqu'à une deuxième section de largeur (23) opposée de la paroi (11), dans lequel des premières ouvertures (25) espacées les unes des autres sont formées dans la première cloison de maintien (17),
∘ une deuxième cloison de maintien (19), espacée de la première cloison de maintien (17) d'une première distance en largeur (ΔB1), qui s'étend parallèlement à la première cloison de maintien (17) depuis la première section de largeur (21) jusqu'à la deuxième section de largeur (23), dans lequel des deuxièmes ouvertures (27) espacées les unes des autres sont formées dans la deuxième cloison de maintien (19), dans lequel chacune des deuxièmes ouvertures (27) est alignée avec une première ouverture (25) respective qui lui est associée, et
∘ une rangée (29) d'éléments d'écartement (33) espacés les uns des autres en formant une pluralité d'espaces libres (31), espacée des cloisons de maintien (17, 19) d'une deuxième distance en largeur (ΔB2) et s'étendant parallèlement aux parois de maintien (17, 19), dans lequel les éléments d'écartement (33) sont disposés de manière décalée par rapport aux ouvertures (25, 27) de telle sorte que chacun des espaces libres (31) soit aligné respectivement avec l'une des premières et des deuxièmes ouvertures (25, 27), de sorte que
∘ à travers respectivement l'un des espaces libres (31), une première ouverture associée (25) et une deuxième ouverture associée (27) définissent une position de maintien (35) pour un corps creux médical (49) qui s'étend dans la direction de largeur (B), et dans lequel
∘ la première distance en largeur (ΔB1) est inférieure à la deuxième distance en largeur (ΔB2).

2. Plateau (1) selon la revendication 1, dans lequel le plateau (1) présente, en tant que l'au moins une structure de maintien, deux structures de maintien (15, 15') disposées l'une à côté de l'autre dans la direction de largeur (B) et séparées l'une de l'autre par une cloison de séparation (37) qui s'étend dans la direction longitudinale (L) depuis la première section de largeur (21) jusqu'à la deuxième section de largeur (23).

3. Plateau (1) selon l'une de revendications précédentes, dans lequel la première cloison de maintien (17) de l'au moins une structure de maintien (15) est espacée d'une cloison frontale (39), choisie parmi une section longitudinale de la paroi (11) et la cloison de séparation (37), d'une troisième distance en largeur (ΔB3).

4. Plateau (1) selon l'une de revendications précédentes, dans lequel les sections de largeur (21, 23) de la paroi (11) présentent chacune un évidement (43) entre la deuxième cloison de maintien (19) et la rangée (29) d'éléments d'écartement (33).

5. Plateau (1) selon l'une de revendications précédentes, dans lequel la plaque de fond (3) présente, dans la zone de la rangée (29) des éléments d'écartement (33), une partie saillante (45), dans lequel les éléments d'écartement (33) sont disposés sur la partie saillante (45).

6. Plateau (1) selon l'une de revendications précédentes, dans lequel les éléments d'écartement (33) sont de forme allongée ou cruciforme en vue du dessus.

7. Plateau (1) selon l'une de revendications précédentes, dans lequel la plaque de fond (3) présente des éléments de pied (47) au niveau de la face inférieure (7) lesquels font saillie depuis la face inférieure (7) et sont disposés de préférence de manière décalée vers l'intérieur d'au moins une épaisseur de paroi de la paroi (11), de préférence de l'épaisseur de paroi, par rapport aux bords extérieurs (9) de la plaque de fond (3).

8. Plateau (1) selon l'une de revendications précédentes, dans lequel le plateau (1) est formé d'une seule pièce et d'un seul matériau, de préférence d'un matériau plastique, le matériau plastique étant de préférence choisi parmi un groupe constitué : d'un matériau plastique autoclavable, d'un matériau plastique lavable en machine, d'un copolymère acrylonitrile-butadiène-styrène, du polycarbonate et du polypropylène.

9. Plateau (1) selon l'une de revendications précédentes en combinaison avec un corps creux médical (49), dans lequel le corps creux médical (49) présente une section cylindrique (51) et une zone de bride (53), dans lequel la zone de bride (53) est disposée entre la première cloison de maintien (17) et la deuxième cloison de maintien (19) et engage par l'arrière de préférence une deuxième ouverture (27) associée au corps creux médical (49) parmi les deuxièmes ouvertures (27), dans lequel la section cylindrique (51) s'étend dans la direction de largeur (B) du plateau (1) à travers la deuxième ouverture (27) et à l'intérieur de l'espace libre (31) associé à la deuxième ouverture (27) associée au corps creux médical (49).

10. Plateau (1) selon la revendication 9 en combinaison avec le corps creux médical (49), dans lequel une tige de piston (55) du corps creux médical (49) s'étend à travers la première ouverture (25) en direction de la cloison frontale (39).
